# EUROPÄISCHE PATENTANMELDUNG

(11) **EP 0 705 573 A2**
(43) Veröffentlichungstag der Anmeldung: **10.04.1996**
(21) Anmeldenummer: 95112714.1
(22) Anmeldetag: 11.08.1995
(51) Int. Cl.: A61B 19/00, A61L 2/26

(54) **Spülkorb für medizinische Instrumente**

(30) Priorität: 30.09.1994 DE 4435223
(71) Anmelder: Richard Wolf GmbH, D-75438 Knittlingen (DE)
(72) Erfinder: Göbel, Jürgen, D-76684 Oestringen (DE)
(74) Vertreter: Wilcken, Thomas, Dipl.-Ing.

(57) **Zusammenfassung**

Der Spülkorb hat wenigstens zwei im Abstand zueinander stehend angeordnete Stützen (2,3), die kammartig mit oben offenen Durchbrechungen (2.8) versehen sind, welche Aufnahmen (2.10 - 2.13) für die Instrumente oder Instrumententeile bilden und diese liegend abstützen. Die Durchbrechungen (2.8) sind mit mehreren übereinander befindlichen Aufnahmen (2.10 - 2.13) versehen, deren lichte Weiten nach oben zur Öffnung der Durchbrechungen (2.8) hin zunehmend größer sind.

## Beschreibung

Die Erfindung betrifft einen Spülkorb für medizinische Instrumente mit mindestens zwei im Abstand zueinander stehend angeordenten Stützen, die kammartig mit oben offenen Durchbrechungen versehen sind, welche Aufnahmen für die Instrumente bilden und diese liegend abstützen.

Solche Spülkörbe dienen zur Aufnahme und Halterung von medizinischen Instrumenten, beispielsweise modularen Zangen, und von Teilen solcher Instrumente während der Desinfektion oder Reinigung in Desinfektions- und Reinigungsautomaten. Die Spülkörbe sind zu diesem Zweck mit Halteelementen oder Stützen für die Instrumente bzw. Instrumententeile versehen und werden beispielsweise in Flüssigkeit durchlassende Siebschalen gestellt (DE-A-42 21 102, DE-C-27 13 094, DE-U-88 12 580, EP-A-0 356 896 und US 4 135 868).

Es kommt vor allem darauf an, daß die zum Reinigen oder Desinfizieren verwendete Flüssigkeit an jedem Bereich des Instrumentariums zur Wirkung kommt und daß außerdem an den gereinigten Teilen nach dem anschließenden Trocknen keinerlei Flüssigkeit mehr anhaftet. Angestrebt wird dies dadurch, daß die Instrumente möglichst punktförmig gehalten werden, wobei bei Instrumenten mit Maulteilen diese offengehalten werden sollen. Weiterhin werden zumindest Hohlinstrumente vorteilhaft in Schräglage abgestützt, um das Ablaufen von Spülflüssigkeit und ein vollständiges Trocknen des Instrumentariums zu gewährleisten.

Diese Erfordernisse sind bei bekannten Spülkörben nicht in idealer Weise erfüllt. So führt einerseits das Bestreben nach möglichst kleinen Kontaktflächen dazu, daß die Instrumente oder Instrumententeile nicht optimal festgehalten werden, oder man nimmt andererseits zugunsten einer sicheren Halterung große Kontaktflächen in Kauf, was aber bewirkt, daß an diesen Flächen weder eine gute Reinigung erzielbar ist noch eine vollständige Trocknung des gereinigten Instrumentes erfolgen kann. Weiter ist festzustellen, daß mit den bekannten Ausführungen eine sichere Offenstellung von Maulteilen nicht erreichbar ist, ohne daß zusätzliche Hilfsmittel, wie etwa Öffnungsfedern, verwendet werden. Schließlich wird bei den bekannten Spülkörben auch nicht der an sich verfügbare Raum für die Aufnahme der Instrumente genutzt, so daß ihre Aufnahmekapazität vergleichsweise gering ist.

Es ist die Aufgabe der Erfindung, einen Spülkorb für Instrumente und Instrumententeile zur Verfügung zu stellen, der bei hoher Aufnahmekapazität dem Instrumentarium während des Reinigungs- und Desinfektionsvorganges einen sicheren Halt bietet und der außerdem gewährleistet, daß nach dem Trocknen keine Restflüssigkeit am Instrumentarium haften bleibt.

Diese Aufgabe wird ausgehend vom eingangs erwähnten Spülkorb erfindungsgemäß dadurch gelöst, daß die oben offenen Durchbrechungen mit mehreren übereinander befindlichen Aufnahmen versehen sind und daß die lichten Weiten der Aufnahmen nach oben zur Öffnung der Durchbrechungen hin zunehmend größer sind. Die Stützen sind als Stege aus einem biegeelastischen Kunststoff ausgebildet, und jede Aufnahme ist durch jeweils zwei seitliche Klemmelemente begrenzt, die einander gegenüberliegende schalenförmige Stützflächen aufweisen und mindestens in ihrem oberen Bereich biegeeleastisch verformbar ausgebildet sind.

Mit einem derart ausgeführten Spülkorb sind neben der Beseitigung der den bekannten Ausführungen anhaftenden, vorstehend beschriebenen Mängeln weitere Vorteile erzielbar. So ist der erfindungsgemäße Spülkorb einfach und kostengünstig herstellbar und das verfügbare Korbvolumen gut ausnutzbar. Weiter können die Teile eines zerlegten Instrumentes geordnet übereinander abgelegt werden, so daß sich bei der Entnahme eine eindeutige Zuordnung der wieder zusammenzubauenden Teile ergibt.

Die erwähnte Biegeelastizität der Klemmelemente und damit die sichere Halterung der Instrumententeile werden dadurch erreicht, daß jedes Klemmelement durch eine äußere Aussparung aus dem Stützenmaterial herausgearbeitet ist, wobei die Stützflächen der jeweils eine Aufnahme begrenzenden Klemmelemente durch eine konische Ausbohrung gebildet sind. Durch diese Maßnahme ist dafür gesorgt, daß bei zuverlässiger Halterung lediglich eine Linienberührung zwischen den Stützflächen und dem gehaltenen Instrumententeil erfolgt.

Weiterhin können etwa bei einer zu desinfizierenden Zange einerseits der Zangenschaft und andererseits die Betätigungsstange für das Maulteil der Zange so in den Aufnahmen festgelegt und eingespannt werden, daß diese Teile bei geöffnetem Maulteilen eingesetzt werden können und die vorher durch Verstellung der Betätigungsstange eingestellte Maulstellung während des Behandlungsvorganges verläßlich beibehalten wird.

Eine gute Kontrolle für den sicheren Sitz und für die erreichte Position des Instrumentes in der jeweiligen Aufnahme ergibt sich durch einen beim Einsetzen fühlbaren Rasteffekt, der dadurch zustandekommt, daß der lichte Abstand zwischen jeweils zwei einander zugeordneten Klemmelementen an deren oberem Bereich kleiner ist als der Durchmesser des einzusetzenden Instrumentes und auch kleiner ist als die Weite der darunter befindlichen Aufnahme.

Die mit den Aufnahmen versehenen Stützen können in einem im wesentlichen quaderförmigen Drahtgestell angeordnet sein, wobei die gewünschte Schräglage des zu reinigenden Instrumentariums durch unterschiedliche Höhenlagen der Stützen erreicht werden kann. Zum Zweck des Austausches der Stützen, beispielsweise um den Spülkorb auf anderskalibrige Instrumente umzustellen oder defekte Stützen zu ersetzen, kann das Drahtgestell mit Führungen versehen sein, auf die die Stützen lösbar und auswechselbar aufgeschoben werden können. Dazu können die Stützen an ihren Stirnseiten mit die Führungen umgreifenden Führungsmitteln versehen und ein die Führungen verbindender Quersteg vorgesehen sein, auf dem sich die Stütze abstützt und der so ihre Höhenposition bestimmt.

Zur vertauschungssicheren Befestigung können die Stützen und die Querstege mit korrespondierenden Durchbrüchen versehen sein, die Druckknopfelemente zur lösbaren Befestigung der Stützen in dem Drahtgestell aufnehmen. Ein wirksamer Schutz der eingelegten Instrumente gegen Beschädigung durch Anstoßen des Drahtkorbes an andere Gegenstände kann dadurch erreicht werden, daß die Langseiten des Drahtgestelles jeweils über die Position der Stützen hinaus verlängert sind und an ihren Enden mit einem Querbügel verbunden sind.

Schließlich kann das Drahtgestell mit nach oben über die Stützen hinausragenden Stützbügeln versehen sein, so daß mehrere in die eingangs erwähnten Siebschalen eingestellte Spülkörbe übereinander angeordnet werden können, ohne daß die eingelegten Instrumente in Mitleidenschaft gezogen werden. Weitere vorteilhafte Merkmale der erfindungsgemäßen Zange sind in Unteransprüchen angegeben.

Die Erfindung wird nachstehend anhand eines in der Zeichnung dargestellten Ausführungsbeispieles näher erläutert. Es zeigen jeweils in perspektivischer Darstellung
- Fig. 1: einen kompletten Spülkorb mit eingelegten Instrumententeilen,
- Fig. 2: einen Spülkorb entsprechend Fig. 1 ohne Stützen und
- Fig. 3: eine Stütze in vergrößerter Darstellung.

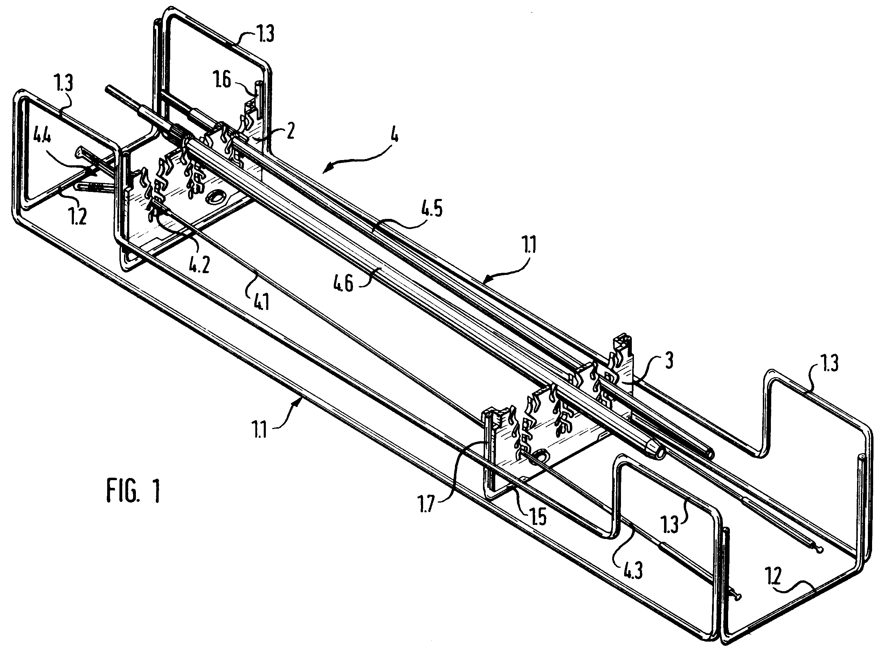

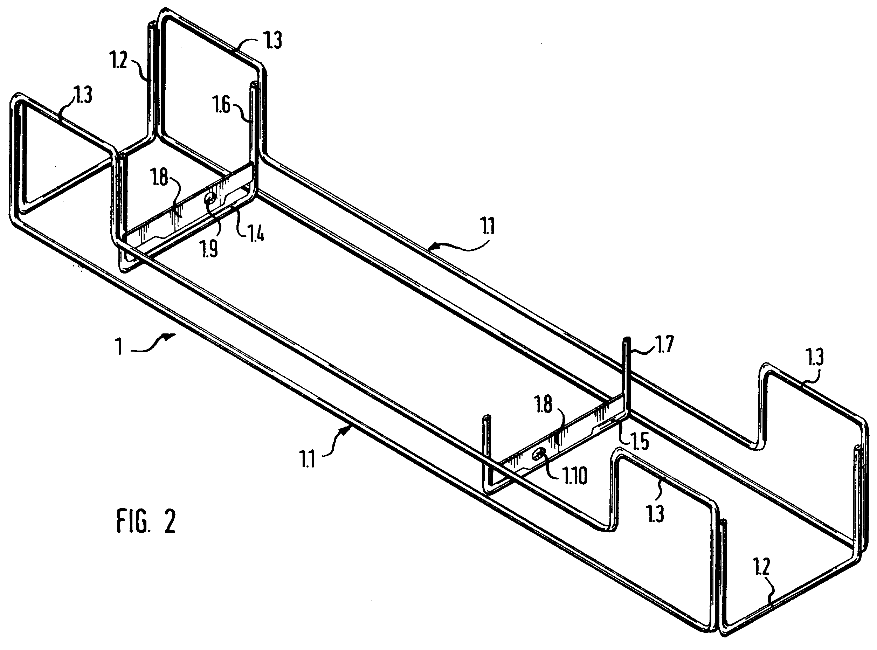

Wie aus den Fign. 1 und 2 erkennbar ist, besteht der Spülkorb aus einem quaderförmigen Drahtgestell 1 mit Stützen 2 und 3 zur Aufnahme von Instrumenten oder Instrumententeilen 4.

Das Drahtgestell 1 ist aus Edelstahldraht gefertigt und weist zwei Seitenteile 1.1 auf, die an ihren Enden durch Querbügel 1.2 unlösbar miteinander verbunden sind und mit diesen einen im wesentlichen rechteckigen Rahmen bilden. Dabei ist die Standfläche durch die unteren Holme der beiden Seitenteile 1.1 und die beiden Querbügel 1.2 bestimmt. Die beiden anderen oberen Holme sind im Mittelteil stufenförmig nach unten abgesetzt, so daß sich nach oben aufragende Stützbügel 1.3 als Träger für einen Zwischenboden ergeben, auf dem ein Spülkorb abgesetzt werden kann.

Im Abstand zu den Querbügeln 1.2 und zueinander beabstandet sind zwei weitere Querbügel 1.4 und 1.5 vorgesehen (Fig. 2). Sie sind U-förmig ausgebildet und derart zwischen den Seitenteilen 1.1 des Drahtgestelles 1 befestigt, daß ihre U-Schenkel nach oben aufragen und Führungen 1.6 und 1.7 für die Stützen 2 bzw. 3 ergeben.

Jeder Querbügel 1.4 und 1.5 weist weiter einen Quersteg 1.8 aus Flachmaterial auf, der jeweils die aufragenden U-Schenkel in der Nähe des Stegteiles des Querbügels 1.4 bzw. 1.5 verbindet und an diesen befestigt ist. Dabei ist der Quersteg 1.8 des Querbügels 1.4 in bezug auf die Standfläche des Drahtgestelles 1 höher angebracht als der des Querbügels 1.5, und beide Querstege weisen jeweils einen Durchbruch 1.9 bzw. 1.10 auf, die unterschiedliche Positionen haben und nicht fluchten.

Jede Stütze 2 und 3 besteht aus einem flächigen, im wesentlichen rechteckigen Steg, welcher bevorzugt aus einem temperaturbeständigen, biegeelastischen und chemisch stabilen Kunststoff im Spritzgußverfahren hergestellt ist. Die Stützen 2 und 3 sind an ihren Schmalseiten 2.1 mit Führungsmitteln 2.2 versehen, die im Bereich der oberen Langseite 2.3 als Führungsschuh ausgebildet und so dimensioniert sind, daß sie auf die Führungen 1.6 bzw. 1.7 aufschiebbar sind. Im Bereich der unteren Langseite 2.4 sind die Stützen abgesetzt und mit einer zu der Langseite parallel verlaufenden Schulter 2.5 versehen. Im Bereich der Abstützung befindet sich ein Durchbruch 2.6 bzw. 2.7, der mit dem Durchbruch 1.9 oder 1.10 der Querstege 1.8 korrespondiert.

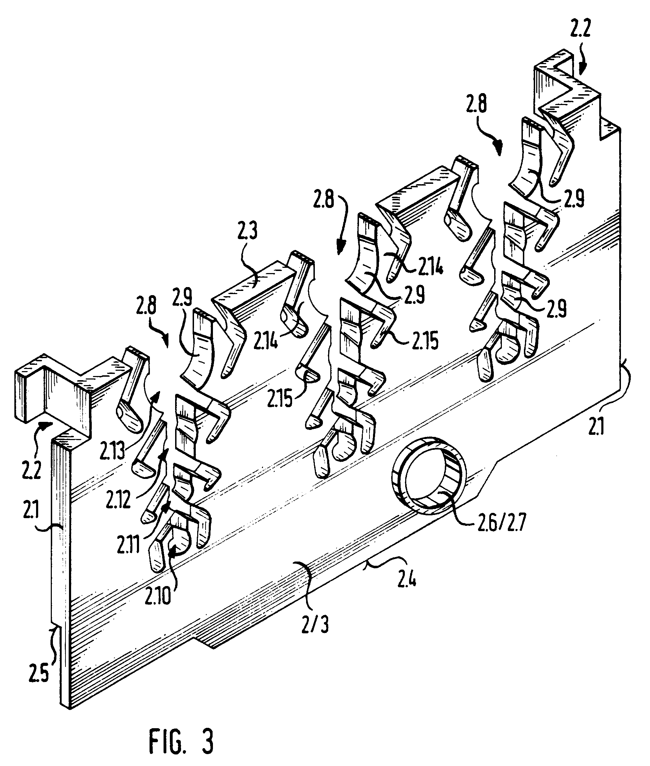

Wie den Fign. 1 und 3 entnehmbar ist, weist jede Stütze 2 und 3 drei nebeneinander angeordnete und nach oben offene Druchbrechungen 2.8 auf. Die Form dieser Durchbrechungen kann man sich dadurch entstanden denken, daß zunächst in das zur Herstellung der Stütze verwendete Stegelement vier konische Bohrungen übereinander angebracht werden, deren Mittelpunkte zu den Schmalseiten 2.1 gleichen Abstand haben und die zur oberen Langseite 2.3 hin zunehmend größeren Durchmesser aufweisen. Diese Bohrungen werden anschließend durch einen von der oberen Langseite 2.3 her geführten symmetrischen Keilschnitt geöffnet, wobei die Breite des Keileinschnittes so gewählt ist, daß von jeder Bohrung einander gegenüberliegende schalenförmige Ausformungen verbleiben. Diese schalenförmigen Ausformungen bilden die Stützflächen 2.9 von in vier Ebenen übereinander angeordneten Aufnahmen 2.10 bis 2.13, die jeweils durch Paare von seitlichen Klemmelementen 2.14 gebildet sind, die durch nach oben offene äußere Aussparungen 2.15 aus dem Stützenmaterial herausgearbeitet sind, so daß die Klemmelemente 2.13 biegeelastisch nach außen ausweichen können.

Zum Zusammenbau des erfindungsgemäßen Spülkorbes werden die Stützen 2 und 3 mit ihren Schmalseiten 2.1 an den Führungen 1.6 bzw. 1.7 entlang zwischen die Seitenteile 1.1 des Drahtgestelles 1 geschoben, so daß schließlich die Führungsmittel 2.2 die Führungen 1.6 bzw. 1.7 umfassen und die Schulter 2.5 auf dem Quersteg 1.8 der Querbügel 1.4 bzw. 1.5 aufsetzt. In dieser Stellung korrespondieren die Durchbrüche 1.9 bzw. 1.10 in den Querstegen 1.8 sowie die Durchbrüche 2.6 bzw. 2.7 in den Stützen 2 und 3 miteinander, sofern die Stützen richtig zugeordnet wurden. Die Stützen 2 und 3 sind nun mittels nicht dargestellter Druckknopfelemente fixierbar, welche in die korrespondierenden Durchbrüche eingedrückt werden.

Die zu reinigenden Teile 4 können nun in die Aufnahmen eingebracht werden, was dadurch geschieht, daß zunächst das Zangenteil 4.1 z. B. eines modularen Zangeninstrumentes in die unterste Aufnahmeebene eingebracht wird. Dabei wird so vorgegangen, daß das Instrumententeil zuerst mit seinem maulseitigen Schaftteil 4.2 in die vordere Stütze 2 und anschließend die Zugstange 4.3 zum Öffnen des Maulteiles 4.4 verstellt und in die hintere Stütze 3 eingerastet wird, so daß das Maulteil 4.4 in Offenstellung verharrt. Sodann können die Schaftteile 4.5 und 4.6 mit größerem Durchmesser in die darüberliegenden Aufnahmeebenen eingebracht werden, wobei die Ebenen in Frage kommen, in denen die Aufnahmen das passende Maß aufweist. Der besseren Übersicht halber zeigt Fig. 1 eine Verteilung der Instrumententeile auf alle drei Durchbrechungen 2.8. Für die Schaftteile 4.5 und 4.6 sind die entsprechenden Aufnahmen in den Stützen 2 und 3 gleich, da diese Teile durchgehend gleichen Durchmesser haben.

Die Stützen 2 und 3 können im Hinblick auf eine eindeutige Zuordnung der zusammengehörenden Teile eines Instrumentes so ausgeführt sein, daß die Aufnahmen für diese Teile in jeweils einer Durchbrechung 2.8, übereinander liegen. Dabei ergibt sich aufgrund der unterschiedlichen Höhenlage der Stützen 2 und 3 eine geneigte Anordnung der Teile, so daß das Abtropfen bzw. Leerlaufen der Instrumententeile nach dem Reinigen begünstigt wird.

Im übrigen besteht die Möglichkeit, den Spülkorb mit mehr als zwei Stützen auszustatten, was vor allem dann angebracht ist, wenn lange Instrumente mehr als zweifach abgestützt werden sollen oder wenn etwa ein vorgegebener Abstand zwischen zwei Stützen größer ist als die Länge der in den Spülkorb einzusetzenden Instrumente.

## Patentansprüche

1. Spülkorb für medizinische Instrumente mit mindestens zwei im Abstand zueinander stehend angeordneten Stützen (2, 3), die kammartig mit oben offenen Durchbrechungen (2.8) versehen sind, welche Aufnahmen (2.10 bis 2.13) für die Instrumente bilden und diese liegend abstützen, dadurch gekennzeichnet, daß die Durchbrechungen (2.8) mit mehreren übereinander befindlichen Aufnahmen(2.10 bis 2.13) versehen sind und daß die lichten Weiten der Aufnahmen nach oben zur Öffnung der Durchbrechungen (2.8) hin zunehmend größer sind.

2. Spülkorb nach Anspruch 1, dadurch gekennzeichnet, daß die Stützen (2, 3) als Stege ausgebildet sind und aus einem biegeelastischen Kunststoff bestehen.

3. Spülkorb nach Anspruch 1 oder 2, dadurch gekennzeichnet, daß jede Aufnahme (2.10 bis 2.13) durch jeweils zwei seitliche Klemmelemente (2.14) begrenzt ist, die einander gegenüberliegende schalenförmige Stützflächen (2.9) aufweisen und mindestens in ihrem oberen Bereich biegeelastisch verformbar ausgebildet sind.

4. Spülkorb nach Anspruch 3, dadurch gekennzeichnet, daß jedes Klemmelement (2.14) durch eine äußere Aussparung (2.15) aus dem Stützenmaterial herausgearbeitet ist.

5. Spülkorb nach einem der Ansprüche 3 und 4, dadurch gekennzeichnet, daß die Stützflächen (2.9) der jeweils eine Aufnahme begrenzenden Klemmelemente (2.14) durch eine konische Ausbohrung gebildet sind.

6. Spülkorb nach einem der Ansprüche 3 bis 5, dadurch gekennzeichnet, daß der lichte Abstand zwischen jeweils zwei einander zugeordneten Klemmelementen (2.14) an deren oberem Bereich kleiner ist als in dem die Aufnahme begrenzenden Bereich.

7. Spülkorb nach einem der Ansprüche 1 bis 6, gekennzeichnet durch ein im wesentlichen quaderförmiges Drahtgestell (1), in welchem die Stützen (2, 3) gegebenenfalls in unterschiedlicher Höhe, angeordnet sind.

8. Spülkorb nach Anspruch 7, dadurch gekennzeichnet, daß das Drahtgestell (1) mit Führungen (1.6, 1.7) zur lösbaren Befestigung der Stützen (2, 3) versehen ist.

9. Spülkorb nach Anspruch 8, dadurch gekennzeichnet, daß jeweils die für eine Stütze (2 oder 3) vorgesehenen Führungen (1.6 bzw. 1.7) durch einen Quersteg (1.8) verbunden sind, auf dem sich die Stütze abstützt und der die Höhenposition der Stütze bestimmt.

10. Spülkorb nach einem der Ansprüche 8 und 9, dadurch gekennzeichnet, daß die Stützen (2, 3) an ihren Stirnseiten mit die Führungen (1.6, 1.7) umgreifenden Führungsmitteln (2.2) versehen sind.

11. Spülkorb nach einem der Ansprüche 7 bis 10, dadurch gekennzeichnet, daß die Stützen (2, 3) gegen Vertauschen gesichert in dem Drahtgestell (1) angeordnet sind.

12. Spülkorb nach einem der Ansprüche 9 bis 11, dadurch gekennzeichnet, daß die Stützen (2, 3) und die Querstege (1.8) mit korrespondierenden Durchbrüchen versehen sind, die Druckknopfelemente zur lösbaren Befestigung der Stützen (2, 3) in dem Drahtgestell (1) aufnehmen.

13. Spülkorb nach einem der Ansprüche 7 bis 12, dadurch gekennzeichnet, daß die Seitenteile (1.1) des Drahtgestelles (1) jeweils über die Position der Stützen (2, 3) hinaus verlängert sind und an ihren Enden jeweils mit einem Querbügel (1.2) verbunden sind.

14. Spülkorb nach einem der Anpsrüche 7 bis 13, dadurch gekennzeichnet, daß das Drahtgestell (1) mit nach oben über die Stützen (2, 3) hinausragenden Stützbügeln (1.3) versehen ist.

15. Spülkorb nach einem der vorhergehenden Ansprüche, dadurch gekennzeichnet, daß jede Stütze (2, 3) mehrere Durchbrechungen (2.8) aufweist.
